# EUROPEAN PATENT APPLICATION

(11) **EP 4 279 606 A1**
(43) Date of publication of application: **22.11.2023**
(21) Application number: 22739450.9
(22) Date of filing: 13.01.2022
(51) Int. Cl.: C12Q 1/06, G01N 33/68

(54) **METHOD FOR PREDICTING RISK FOR THROMBOSIS IN CANCER PATIENT USING SOLUBLE CLEC2**

(30) Priority: 13.01.2021 JP 2021003671
(71) Applicant: LSI Medience Corporation, Tokyo 105-0023 (JP)
(72) Inventor: FUJII Yukihiko, Niigata-shi, Niigata 951-8585 (JP); KAWAMURA Masahide, Tokyo 105-0023 (JP); SHIRAKAWA Kamon, Tokyo 105-0023 (JP)
(74) Representative: Gille Hrabal Partnerschaftsgesellschaft mbB Patentanwälte
(86) International application number: PCT/JP2022/000891
(87) International publication number: WO 2022/154045

(57) **Abstract**

The purpose of the present invention is to develop a biomarker that better reflects thrombus formation in vivo, and to provide a method that enables risk assessment of cancer-associated thrombosis in the perioperative period of cancer patients. The method includes a step of measuring the concentration of soluble CLEC2 in blood collected from the cancer patients.

## Description

### TECHNICAL FIELD

The present invention relates to a method for predicting cancer-associated thrombosis using soluble CLEC2.

### BACKGROUND ART

Thrombus formed in blood vessels is considered to be one of the most important life-threatening factors in a wide range of human diseases, and the risk of thrombus formation is known to be increased in various diseases. The risk of thrombus formation is particularly high in chronic obstructive pulmonary disease (COPD), acute diseases such as infections and sepsis, advanced cancer, pregnancy, nephrotic syndrome, inflammatory bowel disease, and myeloproliferative disorders.

The co-occurrence of thrombosis worsens the prognosis for life. A retrospective clinical study in the United States found a high incidence of thrombosis (16.4%) in 1874 cancer patients (2005-2012). Of these, 2% had a history of thrombosis, but 14.4% are said to have developed the disease within 3 months before the cancer diagnosis.

Pancreatic cancer and brain tumors are especially at risk for thrombosis. The annual incidence of thrombosis was 13.6/1000/year for all cancers, whereas those of thrombosis were 48 persons/1000 persons/year for brain tumors and 58.9 persons/1000 persons/year for pancreatic cancer (Non-patent literature 2). The cause is thought to be the activation of the coagulation system by cancer cells expressing tissue factor (TF) and constantly releasing TF-positive microparticles (MPs) into the blood.

Thus, since the tumor-bearing state is considered one of the thrombotic predispositions, and thrombus is likely to form in the state, venous thromboembolism (VTE) complicated with cancer, so-called cancer-associated thrombosis (CAT), is likely to form. Venous thromboembolism (VTE) means the thrombus formation in a deep vein, and pulmonary artery embolism (PE) means that thrombus in a deep vein releases, is carried into the bloodstream, passes through the right atrium and right ventricle, and causes embolism in the pulmonary artery, resulting in pulmonary thromboembolism. Venous thromboembolism (VTE) is a disease concept that combines deep vein thrombosis (DVT) and pulmonary artery embolism (PE), and in both cases, DVT, especially related to cancer (CAT), has a significant prognostic impact.

The incidence of CAT depends on the type of cancer, and is reported to be higher in cancers of the abdominal cavity and chest, cancers of the brain, and cancers of unknown origin. In addition, the incidence of CAT is also high in cancers with high mortality rates, and it is thought that there is a correlation between the malignancy of cancer and the incidence of CAT.

The highest incidence of CAT is said to occur in the first 3 months after cancer diagnosis, and special attention should be paid to the timing of the start of cancer treatment. It has also been reported that cancer patients who have developed CAT are prone to CAT recurrence and bleeding during anticoagulation therapy. Careful management is required during treatment. Therefore, in the so-called perioperative period, risk management involving many medical personnel is required.

The co-occurrence of venous/arterial thrombosis is common in cancer patients (Non-patent literature 1), and, clinically, progression stage, tumor volume, and length of hospital stay are known to increase the risk of thrombus development.

Trousseau syndrome is also known as an example of hypercoagulability associated with cancer. Trousseau syndrome is defined as "a hypercoagulable state complicated with malignant tumor, and migratory thrombophlebitis associated therewith", and in many cases, malignant tumor is first discovered with the onset of cerebral infarction. Therefore, it is increasingly understood in Japan as "thrombosis associated with DIC complicated with malignant tumor, and systemic (especially cerebral) embolism caused by non-bacterial thrombotic endocarditis (NBTE)". Hereafter, the term CAT is used herein to include Trousseau syndrome.

In addition to leukemia, adenocarcinomas such as lung cancer, pancreatic cancer, stomach cancer, and ovarian cancer (mucin-producing tumor) are by far the most common malignant tumors that cause CAT. Head MRI often shows multiple embolism, and non-infectious thrombotic endocarditis (NBTE) is seen in about half of those patients, but the detection rate by transthoracic echocardiography is low, and transesophageal echocardiography is considered useful for the diagnosis.

The American Society of Clinical Oncology (ASCO) guidelines list increased platelet count, increased white blood cell count, and low hemoglobin as biomarkers to watch for in CAT, but there is no direct diagnostic indicator for CAT (Non-patent literature 3). National and international guidelines describe the management of CAT and provide anticoagulation therapy as initial treatment, and "no treatment" is not an option except for peripheral deep vein thrombosis (DVT), which is discovered incidentally in asymptomatic patients. In the treatment of CAT, it is necessary to take into account the fact that thrombosis can easily form and bleeding can easily occur at the same time, and that the influence of thrombotic predispositions exists not only in venous thrombus but also in arterial thrombus.

Podoplanin has received much attention as a thrombotic predisposition in cancer patients. Podoplanin is a membrane protein highly expressed on the surface of many cancer cells such as squamous cell carcinoma (lung, esophagus, cervix, and the like), mesothelioma, and brain tumor, and is involved in cancer invasion. Kunita et al. found that podoplanin promotes cancer metastasis via platelet aggregation (Non-patent literature 4). Furthermore, podoplanin is reported to be highly expressed in brain tumor and osteosarcoma, and to have high platelet aggregation ability. In addition, a novel anti-podoplanin antibody was found to induce high antitumor effects and inhibit metastasis through cytotoxic activity, indicating that podoplanin is a useful therapeutic target for cancer metastasis.

C-type lectin-like receptor 2 (CLEC2), a receptor for podoplanin, has been identified on platelets as a receptor for the platelet-activating snake venom rhodocytin. The binding of CLEC2 to podoplanin has various pathophysiological roles and has been shown to promote hematogenous metastasis of tumors. Since CLEC2 is expressed almost exclusively in a platelet- and megakaryocyte-specific manner in humans, it is thought to stabilize thrombi by binding homophilically in a platelet activation-dependent manner in flowing blood (Non-patent literature 5).

### CITATION LIST

### PATENT LITERATURE

[Patent literature 1] JP 2008-544224 A
[Patent literature 2] JP 2019-507345 A

### NON-PATENT LITERATURE

[Non-patent literature 1] Trousseau A. Phlegmasia alba dolens. Clinique Medicale de l'Hotel Dieu de Paris, Vol 3. Paris: Bailliere. 1865; 654-712 in French
[Non-patent literature 2] Freesia Horsted et al., PLoS Med. 2012 Jul; 9(7): e1001275
[Non-patent literature 3] Lyman GH et al., J Clin Oncol 31(17):2189-2204, 2013
[Non-patent literature 4] Kunita A et al., Am J Pathol. 170:1337-1347 (2007)
[Non-patent literature 5] Inoue K et al., Jpn J Thromb Hemost. 22(6):348-362(2011)

### SUMMARY OF INVENTION

### TECHNICAL PROBLEM

The prognosis of extensive thrombosis is extremely poor, and early and accurate diagnostic methods are essential to provide appropriate treatment that significantly improves mortality. Therefore, even in cancer patients, it is necessary to manage the perioperative hypercoagulable state of blood sufficiently to assess the risk of thrombosis, and to strive for prevention and early treatment.

Known methods for diagnosis and risk stratification of venous thromboembolism include a method that combines not only coagulation- and hemostasis-related markers but also multiple markers such as blood pressure regulation, inflammation, myocardial damage, and lung damage (Patent literature 1), and a method that combines a measurement of D-dimer and a measurement of coagulation factor activity using fibrin formation as an indicator (Patent literature 2). However, none of these markers is easy to implement, and none of them can be said to accurately assess the risk of thrombus formation. Therefore, there are no markers that can be used for risk assessment and monitoring to meet the needs of clinical practice.

Therefore, a purpose of the present invention is to develop a biomarker that better reflects thrombus formation in vivo, and to provide a method that enables risk assessment of CAT in the perioperative period of cancer patients.

### SOLUTION TO PROBLEM

The present inventors have conducted intensive studies to solve the problem. As a result, it was found that the concentration of soluble CLEC2 (hereinafter sometimes referred to as sCLEC2) in the plasma of cancer patients was significantly correlated with CAT, leading to the completion of the present invention.

Furthermore, by measuring sCLEC2 concentrations in cancer patients over time and observing their fluctuations, the present inventors could establish a method for early, simple, and accurate risk assessment of CAT. The present inventors validated the method using several dozen patient samples and showed that the method is useful for CAT risk prediction and monitoring in patients with pancreatic cancer and patients with brain tumor.

The present invention provides the following:
[1] A method for assessing a risk of cancer-associated thrombosis in a perioperative period of a cancer patient, comprising the step of measuring a concentration of soluble CLEC2 in blood collected from the cancer patient.
[2] The method of [1], comprising:
   (1) providing a sample from a patient who may have cancer-associated thrombosis, or a patient who has been diagnosed with cancer-associated thrombosis;
   (2) determining a concentration of soluble CLEC2 in the sample; and
   (3) correlating the soluble CLEC2 concentration with a presence or absence of cancer-associated thrombosis in the patient, or with likelihood of outcome.
[3] The method of [1] or [2], wherein the step of correlating the soluble CLEC2 concentration with a presence or absence of cancer-associated thrombosis in the patient, or with likelihood of outcome comprises a step of assessing whether the patient is at risk of cancer-associated thrombosis based on a change in the soluble CLEC2 concentration.
[4] The method of any one of [1] to [3], wherein at least one of coagulation and hemostasis-related marker is used in addition to the soluble CLEC2 concentration.
[5] The method of any one of [1] to [4], wherein a value obtained by dividing the soluble CLEC2 concentration by a platelet count is used instead of the soluble CLEC2 concentration.
[6] A method for predicting efficacy determination of an antiplatelet agent in a method for assessing a risk of cancer-associated thrombosis in a perioperative period of a cancer patient, by providing samples derived from the cancer patient from preoperative period to 30 days postoperatively over time, and monitoring risk assessment continuously.
[7] The method of any one of [1] to [6], wherein the cancer is selected from the group consisting of pancreatic cancer, squamous cell carcinoma (lung, esophageal, cervix, and the like), mesothelioma, brain tumor, advanced cancer, and myeloproliferative disease.
[8] The method of any one of [1] to [7], wherein the cancer-associated thrombosis is Trousseau syndrome.

### ADVANTAGEOUS EFFECTS OF INVENTION

The method of the present invention, measuring sCLEC2 concentrations present in the blood of cancer patients, enables early, simple, and accurate risk assessment of CAT. Furthermore, it is expected to improve the prediction accuracy of perioperative monitoring for the development of CAT, including the period after surgery or chemotherapy in cancer patients, compared to conventional tests using blood markers or platelet aggregation tests.

### BRIEF DESCRIPTION OF THE DRAWINGS

[Fig. 1] Figure 1 is a standard curve prepared using a hsCLEC2 protein as a standard.
[Fig. 2] Figure 2 is a graph in which the concentrations of sCLEC2 in plasma are compared between pancreatic cancer patients and healthy subjects.
[Fig. 3] Figure 3 is a graph in which the concentrations of sCLEC2 in plasma are compared between the presence and absence of blood abnormalities.
[Fig. 4] Figure 4 is a graph in which the concentrations of sCLEC2 and D-dimer in plasma are compared between the presence and absence of blood abnormalities.
[Fig. 5] Figure 5 is a graph in which the concentrations of sCLEC2 in plasma are compared between brain tumor patients with or without DVT.
[Fig. 6] Figure 6 is a graph in which the relationship between the presence or absence of DVT development and the C2PAC value.
[Fig. 7] Figure 7 is a graph in which the temporal changes of various markers in the perioperative case of a glioblastoma patient.

### DESCRIPTION OF EMBODIMENTS

Hereinafter, the embodiments of the present invention will be explained in detail, but the embodiments of the use are not limited thereto.

For example, the present invention includes:
a method of assessing the risk of CAT, characterized by measuring (or determining) the concentration of soluble CLEC2 (or a value obtained by dividing the concentration of soluble CLEC2 by platelet count) in a sample;
a method of assisting in the risk assessment of CAT, characterized by measuring (or determining) the concentration of soluble CLEC2 (or a value obtained by dividing the concentration of soluble CLEC2 by platelet count) in a sample;
a method of measuring (or determining) the concentration of soluble CLEC2 (or a value obtained by dividing the concentration of soluble CLEC2 by platelet count) in a sample for the risk assessment of CAT;
an in vitro method of assessing the risk of CAT, characterized by measuring (or determining) the concentration of soluble CLEC2 (or a value obtained by dividing the concentration of soluble CLEC2 by platelet count) in a sample;
use of an antibody capable of detecting the concentration of soluble CLEC2 in the manufacture of a kit for risk assessment of CAT; and
a method of measuring (or determining) the concentration of soluble CLEC2 (or a value obtained by dividing the concentration of soluble CLEC2 by platelet count) in a sample to provide information necessary for the risk assessment of CAT.

The term "cancer" as used herein means a group of diseases that cause uncontrolled cell proliferation, i.e., invasion and spread of cells from the site of origin, i.e., the primary site, to other parts of the body, and is not limited to cancers arising from epithelial cells. It is assumed to be suitable in cancers where podoplanin or its receptor, CLEC2, is found to be involved, as described below. In general, cancers are classified according to the organ, tissue, shape, and the like in which they arise. The cancers include squamous cell carcinoma, which is a malignant proliferation of cells called epidermal keratinocytes in the epidermis; basal cell carcinoma, which is a cancer arising from cells that constitute the basal layer (which is a lower layer), hair follicles, or the like; myeloproliferative diseases; and the like. In the case of classification by organ, for example, brain tumor, tongue cancer, laryngeal cancer, thyroid cancer, esophageal cancer, stomach cancer, colon cancer, hepatocellular cancer, gallbladder cancer, bile duct cancer, pancreatic cancer, lung cancer, mesothelioma, breast cancer, ovary cancer, cervix cancer, uterine cancer, renal cell cancer, renal pelvis ureter cancer, prostate cancer, bladder cancer, skin cancer, bone and soft tissue tumor, leukemia, malignant lymphoma, childhood cancer, and the like, may be exemplified, but are not limited thereto. In addition, advanced cancers are also included.

The term "cancer-associated thrombosis (CAT)" as used herein is understood as a generic term for cancer-related thrombosis. Thrombosis that may occur in cancer patients includes stagnation of blood flow, dehydration, and bed rest due to cancer; venous thromboembolism associated with cancer itself, such as enhancement of coagulation system by cancer cells; vasculopathy or enhancement of coagulation system associated with chemotherapy; vascular injury by catheters; retention; portal retention associated with portal hypertension; venous thromboembolism associated with cancer treatment, such as portal vasculopathy; previous venous thromboembolism; obesity; advanced age; prolonged bed rest; paraplegia; cast immobilization; thrombotic predispositions (antithrombin deficiency, protein C/S deficiency, antiphospholipid antibody syndrome, and the like); estrogen therapy; varicose veins; congestive heart failure; respiratory failure; venous thromboembolism caused by additional risks other than cancer, such as severe infection; and the like, but it includes all of them.

The term "Trousseau syndrome", which is positioned as an embodiment of CAT, is not limited to conditions presenting stroke symptoms caused by hypercoagulability in CAT, but means thrombosis associated with DIC complicated with malignant tumor, and systemic embolism caused by non-bacterial thrombotic endocarditis (NBTE).

The term "perioperative period" as used herein means a series of periods after the decision of surgery including pre- and postoperative periods from outpatient to hospitalization, anesthesia and surgery, postoperative recovery, discharge and reintegration into society.

The term "CLEC2" as used herein is a platelet-activating receptor belonging to a C-type lectin family, which is usually present on the membrane of platelets and is released into the blood upon platelet activation. The term "soluble CLEC2 (sCLEC2)" as used herein means CLEC2 or CLEC2-derived molecules that are released from such platelets and detected in blood (or buffer if incubated in buffer).

sCLEC2 includes proteins with molecular weights of approximately 40 kDa, approximately 32 kDa, approximately 25 kDa, and the like in SDS-polyacrylamide gel electrophoresis (SDS-PAGE) under reducing conditions. The proteins with molecular weights of approximately 40 kDa and approximately 32 kDa are present on the membrane surface of platelets, and it is presumed to be released in the form of being included in microparticles produced upon platelet activation. These are thought to have sugar chains attached to them. On the other hand, the protein with a molecular weight of approximately 25 kDa are thought to be cleaved by proteases and released from platelets upon platelet activation. In the present invention, the amount of sCLEC2 as described above is measured. sCLEC2 can be detected together as proteins with molecular weights of approximately 40 kDa, approximately 32 kDa, and approximately 25 kDa, or it can be detected only as a protein with a molecular weight of approximately 25 kDa.

As the concentration of sCLEC2 used in the present invention, the concentration of sCLEC2 may be used alone, or the value obtained by dividing the concentration of sCLEC2 by platelet count may be used. In the present specification, unless otherwise specified, the concentration of sCLEC2 is interpreted to include both the case of using the sCLEC2 concentration and the case by dividing the sCLEC2 concentration by platelet count.

Samples for the measurement are preferably derived from humans, but samples derived from non-human animals may be used for the purpose of understanding the clinical conditions or the like of experimental animals. Such experimental animals are not limited, but include, for example, a guinea pig, a rat, a mouse, a chinchilla, and the like.

The method of the present invention is also suitable for testing for thrombotic and hemostatic diseases. The term "hemostasis" as used herein means that platelets and coagulation factors work together to effectively and appropriately stop blood flow or bleeding. The term "thrombotic and hemostatic diseases" include, but are not limited to, conditions and diseases involving excessive bleeding and abnormal blood coagulation. In particular, it can be suitably used to predict the risk of venous thromboembolism (VTE) and cancer-associated thrombosis (CAT), which is VTE complicated with cancer.

For example, if the sCLEC2 concentration is higher than that of normal persons or a non-thrombotic and hemostatic disease group, it can be said that the possibility of having a CAT thrombotic and hemostatic disease or the risk of contracting such a disease is high. Based on such a comparison, the sCLEC2 concentration can be compared between pre- and postoperative periods and used as a risk prediction for thrombosis.

For example, if the sCLEC2 concentration is measured in patients with pancreatic cancer or brain tumors, and the ratio of sCLEC2 concentration is high, it can be judged that platelet activation in vivo is occurring, and may be used to administer antiplatelet agents such as aspirin as primary prophylaxis. Furthermore, in patients taking antiplatelet agents such as aspirin and clopidogrel, the sCLEC2 concentration can be measured, and if the value is high, it is possible to consider increasing the dose of the antiplatelet agent, changing to a different type of antiplatelet agent, or administering an additional agent.

A method for detecting the presence of sCLEC2 is not limited, but immunological methods using an antibody that recognizes sCLEC2 (hereinafter sometimes referred to as "anti-sCLEC2 antibody") are preferred. As the methods in which the protein is immunologically detected, any of the following methods which are known and commonly used can be used: immunoassays using labeled antibodies such as enzyme-linked immunoassay (ELISA), chemiluminescence immunoassay, fluorescent antibody method, radioimmunoassay, and immunochromatography, or Western blotting, latex agglutination, immunoturbidimetric method, and the like. Among these methods, immunoassays using labeled antibodies is preferably used from the viewpoints of simplicity of operation and accuracy of measurement. For intraoperative diagnosis, chemiluminescence immunoassay or immunochromatography is especially preferred, since rapid results are desired.

A sample is collected from a subject (especially a patient), for example, with a blood collection tube for plasma collection. Although EDTA-containing blood collection tubes are suitable for platelet count, heparin- or citric acid-containing tubes can also be used, and those skilled in the art can select an appropriate tube from among these. Samples for the measurement of sCLEC2 concentration in plasma and the measurement of platelet count may be obtained in a single blood collection tube, or in separate tubes if they are collected at the same time. The sCLEC2 concentration in plasma is measured by centrifuging plasma, for example, at 2000g for approximately 20 minutes, but the centrifugation conditions are not limited to this and whole blood may also be used. The following explanation is based on, but not limited to, the measurement of the sCLEC2 concentration in plasma. Whole blood containing an anticoagulant such as EDTA is used for the platelet count in blood.

For the correlation between the measured sCLEC2 concentration in cancer-patient-derived samples and the presence or absence of CAT, or the likelihood of outcome, a threshold value may be appropriately set and used based on the comparison of sCLEC2 concentrations in cancer-patient-derived samples and those in healthy-subject-derived samples, or the risk assessment of CAT may be performed when significant changes in sCLEC2 concentrations are detected from the temporal record of sCLEC2 concentrations, such as comparison between preoperative and postoperative sCLEC2 concentrations or comparison between the first postoperative day and several days after surgery in the same patient.

When the sCLEC2 concentration is divided by platelet count to predict the risk of CAT, the platelet count is usually measured using an automated hemocytometer (blood count meter), but can also be counted using a hemocytometer plate and a microscope.

The sCLEC2 concentration in plasma is expressed in, for example, pg/mL, and the platelet count in blood is expressed in, for example, 1000 platelets/mm³, and sCLEC2 concentration/platelet count is calculated. The concentration of sCLEC2 used herein can be expressed in ng/mL, ng/L, or any other arbitrary unit, and the platelet count can be expressed in 10000 platelets/mm³ or any other arbitrary unit, but a uniform unit should be used for comparison. By using various units, sCLEC2 concentration/platelet count can take various values, but they are essentially the same concept.

The calculation of the ratio is performed using measured values from a clinical analyzer that measures the sCLEC2 concentration and measured values from a blood count meter that measures the platelet count. Although it is preferable in daily practice to perform this calculation automatically on a hospital laboratory system, a hospital system, or an electronic medical record system that is connected to both analyzers, it is also possible to construct a system that connects the data of the two analyzers, or to construct a machine that can simultaneously measure the sCLEC2 concentration and platelet count. Furthermore, the ratio may also be computed manually using both data.

Furthermore, the correlation between the sCLEC2 concentration in plasma or sCLEC2 concentration/platelet count and the degree of platelet activation or various diseases may be used, for example, as a threshold for judgment, or as original data or statistical processing data to calculate the threshold for judgment.

sCLEC2 is released into the blood upon platelet activation. Conventional platelet activation markers, such as PF4 and βTG, have problems, because the physical pressure of blood sampling stimulates the granules and causes nonspecific release. On the other hand, sCLEC2 is a signal transduction-dependent release mechanism that triggers platelet activation, and may be a more accurate marker for platelet activation in vivo. In addition, CLEC2 may be a specific marker with few false positives because its expression is almost limited to the platelet and megakaryocyte system in humans. Therefore, measurement of sCLEC2 enables early diagnosis of platelet activation status, and can be used as a method to predict the risk of thrombosis.

Since the sCLEC2 concentration in plasma tends to be higher in individuals with high platelet counts and lower in those with low platelet counts, if the sCLEC2 concentration in plasma is affected by the platelet count in the blood and does not necessarily represent platelet activation, the value obtained by dividing the sCLEC2 concentration in plasma by the platelet count in the blood may be used, taking advantage of the positive correlation between the sCLEC2 concentration and the platelet count in the blood.

The diagnosis of thrombotic diseases by dividing the sCLEC2 concentration in plasma by the platelet count in the blood and calculating the amount of sCLEC2 released per platelet as an index is preferable because it is possible to evaluate the degree of platelet activation without depending on the number of platelets in the blood. Specifically, for example, the sCLEC2 concentration in plasma is expressed in pg/mL (A), the platelet count in the blood is expressed in 1000 platelets/mm³ (B), and the number obtained by dividing A by B can be used as an index of platelet activation.

In the case of surgically treated cancer patients, it is possible to predict the risk of postoperative thrombosis by collecting blood over time, measuring the sCLEC2 concentrations in plasma, and observing the changes in the concentrations. For example, by appropriately setting the period of sample collection according to the background information of each patient, it is possible to obtain a more detailed profile, observe the conditions, and assess the risk. For patients who are observed to have a marked increase over the preoperative period, it can be used not only to predict that they are at risk for CAT, but also as a method of monitoring their conditions after drug administration, even in patients diagnosed with CAT.

CAT is considered to have a high recurrence rate, and long-term anticoagulation therapy is considered to be necessary in many cases. However, anticoagulant therapy for CAT may increase the risk of bleeding, and anticancer drugs may cause hematologic toxicity, which decreases white blood cells (especially neutrophils), red blood cells, and platelets. Therefore, since the long-term treatment plan for CAT must be decided in consideration of bleeding risk and prognosis, although it is considered difficult to determine the duration of anticoagulation therapy, no clear standard has been established for the anticoagulation therapy. Therefore, the present invention may be used to assess the risk of CAT in patients, which may assist in making appropriate decisions on the treatment plan.

The frequency of concentration measurements used for monitoring may be appropriately set according to the background information including the treatment history and the like of individual patients and the treatment plan. In such cases, risk assessment can be performed by collecting samples at regular intervals from preoperative period to 30 days postoperatively. However, since the situation is expected to differ depending on the patient's background, the monitoring period and timing can be appropriately set, for example, by using periodic observation during the period from 7 to 10 days postoperatively, which generally requires special attention in terms of the occurrence of CAT.

The present invention may be used in combination with biomarkers currently reported to be useful for risk assessment of CAT in order to assist risk assessment by the sCLEC2 concentration. The use of these biomarkers in combination with sCLEC2 is preferred because it allows for a more accurate risk assessment of CAT. For example, D-dimer and the like, may be used for postoperative monitoring applications similar to the present invention. However, as in the cases of the Examples described below, the inventors found that in some cases of pancreatic cancer, although the sCLEC2 concentration fluctuated in cases diagnosed with CAT, the D-dimer did not fluctuate at all and the risk of CAT could not be assessed in some cases. This may depend on the mechanism of thrombus formation. That is to say, monitoring over time of fibrin-based thrombus formation by D-dimer and platelet-based thrombus formation by sCLEC2 may allow more accurate risk assessment of CAT. In other words, platelet-dominant thrombus is predictable.

The present invention may be used in combination with other findings such as image evaluation. In general, in clinical practice, diagnosis is made by imaging diagnosis for patients suspected of having CAT, and therefore, the use of these information in combination is expected to improve the accuracy of the diagnosis, and is therefore preferred.

In general, as treatments for thrombosis, in the case of thrombi in the arteries where activation and aggregation of platelets proceed and it promotes coagulation, such as myocardial infarction, atherothrombotic stroke, and arteriosclerosis obliterans, anti-platelet drugs such as aspirin is selected, whereas in the case of thrombi where the coagulation system is activated and coagulation proceeds in the veins and atria, such as deep vein thrombosis, atrial fibrillation, cardiogenic cerebral embolism, and pulmonary embolism, a treatment with anticoagulants such as warfarin or DOACs is selected. In addition, although CAT, a cancer-associated thrombosis, is assumed as the form of use of the present invention, anticoagulants are currently used as the standard treatment. The present invention may enable the selection of antiplatelet agents by assessing the risk of platelet-based thrombosis. Therefore, monitoring the sCLEC2 concentration in postoperative patients using the present invention is of great significance, because it enables rapid, reliable, and cost-effective stratification of postoperative risk, the need for postoperative care or means of treatment by monitoring can be reliably assessed and estimated. For example, the method of the present invention can be of great benefit because it enables us to determine the efficacy of treatment and to predict the risk quickly with reliable accuracy, and to select drugs with lower bleeding risk.

### EXAMPLES

The present invention will now be further illustrated by, but is by no means limited to, the following Examples.

### «Example 1: Measurement of sCLEC2 in human plasma»

The sCLEC2 concentrations in plasma were measured in accordance with Example 6 of Japanese Patent No. 4,961,595.

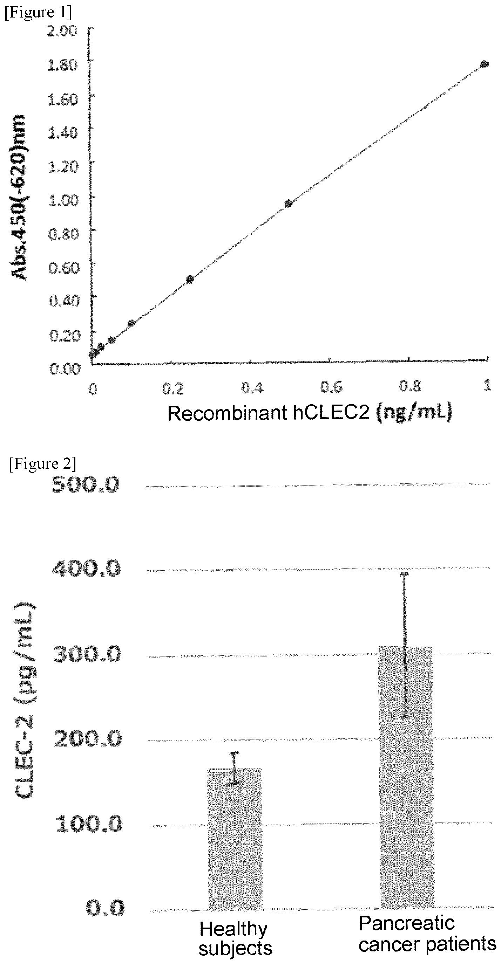

A sandwich ELISA system was constructed using mouse anti-human sCLEC2 antibodies. More particularly, a 1-11D5 antibody (F(ab)'₂) purified with a 0.05 mol/L carbonate buffer (pH 9.5) was diluted to 10 µg/mL, and added to an immunoplate (Maxisorp; NUNC) at 100 µL/well. After overnight reaction at 4°C, each well was washed with phosphate buffered saline (PBS) containing 0.05% Tween-20 three times, and 200 µL of PBS containing 1% bovine serum albumin (BSA) was added to each well for blocking. Next, a standard dilution series of human sCLEC2 (hsCLEC2) protein used as a standard was prepared using 10% SuperBlock (Thermo Fisher Scientific), 0.1% sodium octanoate, and 0.14 mol/L sodium chloride/phosphate buffer (PB). Human plasma was diluted at least 5-fold with the same buffer. Each was added at 100 µL/well, reacted at 37°C for 1.5 hours, and washed three times in the same manner. Next, a prepared biotin-labeled 3-11E6 antibody (F(ab)'₂-biotin) was diluted with 10% SuperBlock, 0.1% sodium octanoate, and 0.14 mol/L sodium chloride/PB to 1.0 µg/mL, and added to each well at 100 µL/well. After a reaction at 37°C for 1 hour, each well was washed three times in the same manner. Next, AMDEX streptavidin-conjugated horseradish peroxidase (GE Healthcare) was diluted with 10% SuperBlock, 0.1% sodium octanoate, and 0.14 mol/L sodium chloride/PB, and added to each well at 100 µL/well. After a reaction at 37°C for 1 hour, each well was washed five times in the same manner, and a 3,3',5,5'-tetramethylbenzidine (TMB) solution was added to each well at 100 µL/well. After a reaction at room temperature for approximately 20 minutes, the reaction was stopped with 1 mol/L sulfuric acid solution. Absorbance at 450 nm (-620 nm) was measured with a plate spectrophotometer (BIO-TEK INSTRUMENTS/EL312e). Figure 1 shows a standard curve prepared using the hsCLEC2 protein as a standard.

### «Example 2: Measurement of sCLEC2 in plasma samples from pancreatic cancer patients»

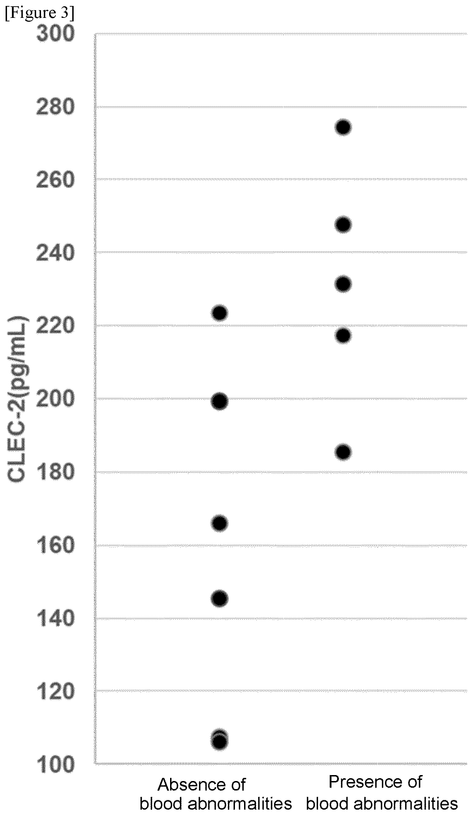

Plasma from 14 pancreatic cancer patients and 10 healthy subjects purchased from BiolVT (Westbury, NY) was diluted with the buffer and measured by the method of Example 1. Table 1 shows the obtained results of the pancreatic cancer patients. Furthermore, Figure 2 shows the mean ± SE of sCLEC2 concentrations in healthy subjects and pancreatic cancer patients. The sCLEC2 concentration was significantly elevated (p<0.05) compared to the healthy subjects. It was revealed from this that platelets are activated by the influence of cancer in pancreatic cancer patients. Next, when the pancreatic cancer patients were classified according to the presence or absence of blood abnormalities, as shown in Figure 3, sCLEC2 values were higher in patients with blood abnormalities than in those without blood abnormalities. Among them, patients diagnosed with an abnormal coagulation profile showed 1382.2 pg/mL, which was approximately 8 times higher than the average value in healthy subjects, and it was revealed that patients with high thrombosis risk showed high values.

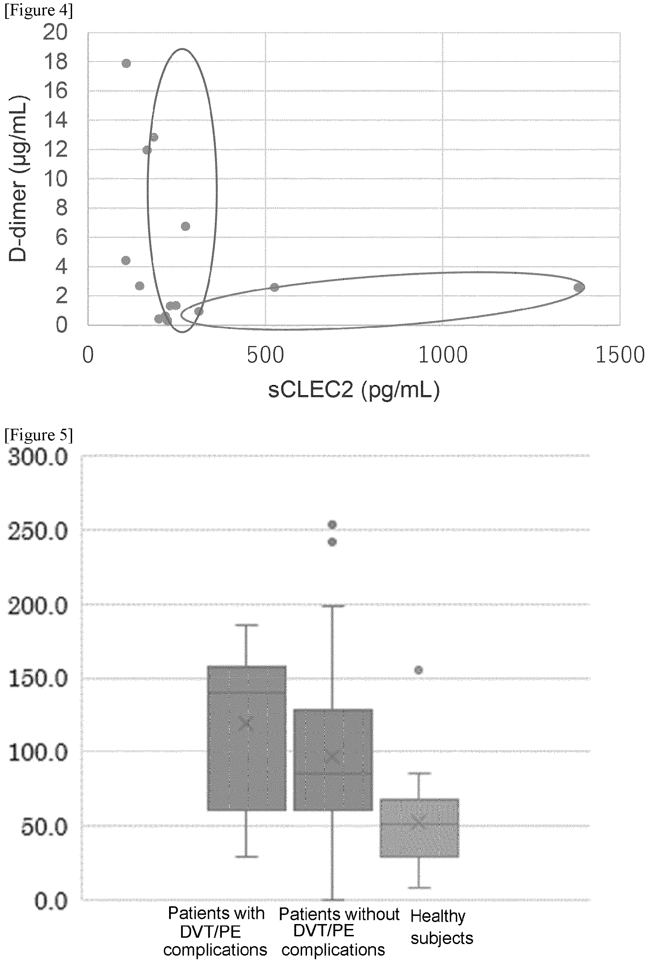

Monitoring of VTE is generally performed by D-dimer in addition to imaging evaluation. Therefore, D-dimers in plasma samples of 14 pancreatic cancer patients purchased from BiolVT were measured with an LPIA Genesis D-dimer reagent (LSI Medience) and compared with the sCLEC2 concentrations measured in Example 2 (Figure 4). As a result, we observed two groups of patients, one in which D-dimer was elevated relative to sCLEC2, and the other in which sCLEC2 was elevated relative to D-dimer. Thrombosis is classified into venous thrombosis and arterial thrombosis. Venous thrombus is considered to be a thrombus composed mainly of fibrin, while arterial thrombus is considered to be a thrombus composed mainly of platelets. While D-dimer, fibrin degradation products, is a commonly used biomarker for venous thrombosis composed mainly of fibrin, there is no such commonly used biomarker for arterial thrombosis such as Trousseau syndrome composed mainly of platelets. The fact that a group of patients with elevated sCLEC2 without elevated D-dimer was observed in this Example indicates that sCLEC2 captures the clinical condition of arterial thrombosis composed mainly of platelets, and that sCLEC2 is useful for testing for Trousseau syndrome, which is not detectable by conventional D-dimer testing.

**[Table 1]**

| Sample No. | Diagnosis | Blood abnormalities | Stage | Metastasis | sCLEC2 pg/mL |
|---|---|---|---|---|---|
| 316362 | Pancreatic cancer | Absence | 4 | Liver | 223.7 |
| 320201 | Pancreatic cancer, Aplastic anemia | Presence | 4 | Intestine, Lymph | 274.4 |
| 586265 | Pancreatic cancer, Aplastic anemia, Hypertension, Thyroid gland abnormalities, Lung diseases | Presence | 4 | Peritoneal nodule | 185.5 |
| 520928 | Pancreatic cancer, Aplastic anemia, Hypertension, Type 2 diabetes | Presence | 3 | Lymph | 217.4 |
| 542895 | Pancreatic cancer | Absence | 4 | Liver | 107.6 |
| 295845 | Pancreatic cancer, Thyroid gland abnormalities, Arthrosis | Absence | 3 | Absence | 525.8 |
| 566261 | Pancreatic cancer, Asthma, Anorexia Nervosa | Absence | 3 | Absence | 312.2 |
| 586412 | Pancreatic cancer, Liver cancer, Bone cancer, Asthma | Absence | 4 | Liver, Spinal cord | 166.2 |
| 588191 | Pancreatic cancer, Allergic rhinitis, Thoracic aortic aneurysm, Gallbladder stones, Hypokalemia, Anemia, Elevated liver function tests, Hyperbilirubinemia | Presence | 1 | Absence | 231.4 |
| 496183 | Pancreatic cancer, Hypertension (HTN), Lupus, Arthritis, Fibromyalgia syndrome (FMS), Herniated disc, Gastroesophageal reflux disease (GERD) | Absence | 3 | Presence | 106.3 |
| 521189 | Pancreatic cancer, Type 2 diabetes | Absence | 4 | Liver | 145.5 |
| 560307 | Pancreatic cancer, Depression, Hypokalemia, Hypothyroidism, Type 2 diabetes, Liver abscess | Absence | 2 | Absence | 199.5 |
| 586341 | Pancreatic cancer, Type 2 diabetes, Thrombocytopenia | Presence | 2 | Absence | 247.8 |
| 583875 | Pancreatic cancer, Abnormal coagulation profile, Anemia | Presence | 3 | Absence | 1,382.2 |

### «Example 3: Measurement of sCLEC2 in plasma samples from brain tumor patients»

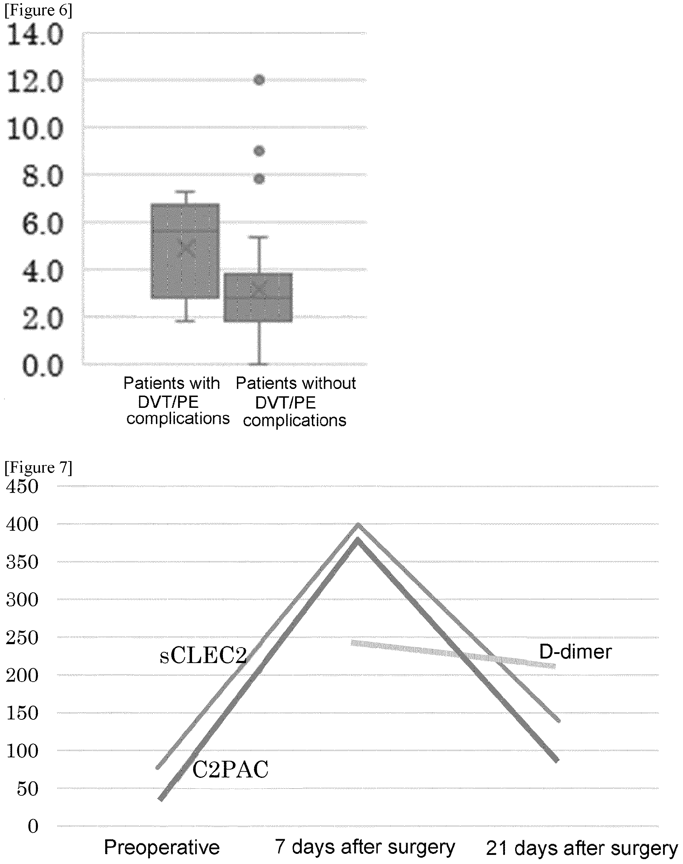

Plasma samples were collected from patients with brain tumors, mainly malignant glioma, admitted to the neurosurgery department 7 to 10 days after surgery, and from healthy subjects who gave consent, and the sCLEC2 concentrations were measured by the method of Example 1. The results of the samples obtained are shown in Tables 2 and 3. Figure 5 shows the sCLEC2 concentrations in patients with DVT/PE complications (n=10), patients without DVT/PE complications (n=48), and healthy subjects (n=15). The sCLEC2 concentrations tended to be higher in patients with DVT/PE than in those without DVT/PE complications 7 to 10 days after surgery (p=0.239). Furthermore, the results of the mean, standard deviation, and median values for patients with DVT/PE complications and those without DVT/PE complications are shown in Table 4.In connection with this, DVT was screened by the D-dimer value and clinical symptoms, and was identified at an average of 10 days after surgery. This indicates that platelets may be activated in patients with DVT/PE in the perioperative period of brain tumors. Furthermore, since CLEC2 is expressed on platelets and may be affected by the platelet count, when a value obtained by dividing the sCLEC2 concentration by the platelet count (a value obtained by dividing the sCLEC2 concentration (pg/mL) by the platelet count (1000 cells/µL); hereinafter sometimes referred to as C2PAC) was used to compare the two groups, a significant increase was observed in patients with DVT/PE complication compared to those without DVT/PE complication (p=0.032; Table 2, Table 3, and Figure 6).

Monitoring of DVT is generally performed by D-dimer in addition to imaging evaluation. Therefore, the D-dimer concentration, the sCLEC2 concentration, and the C2PAC value were evaluated in a patient (glioblastoma, 71 years old, female) whose blood samples could be taken over time (Fig. 7). The patient was identified as having DVT on the seventh day after surgery, and anticoagulation therapy was started. The sCLEC2 and C2PAC values were maximal on day 7 when the patient was identified as having DVT, and then, it was thought that the values were decreased progressively with the effect of anticoagulation therapy. On the other hand, in addition to the time of DVT identification, the D-dimer remained relatively high 14 days after the start of anticoagulation therapy, but did not change significantly. The sCLEC2 and C2PAC values were more sensitive to the therapy, and it was presumed in this case that the sCLEC2 and C2PAC values were the most useful for monitoring the therapeutic effect of the anticoagulation therapy.

**[Table 2]**

| Case No. | DVT/PE | sCLEC2 after surgery pg/mL | C2PAC value |
|---|---|---|---|
| A | With | 110 | 3.9 |
| B | With | 186 | 7.1 |
| C | With | 158 | 5.9 |
| D | With | 140 | 5.7 |
| E | With | 139 | 6.6 |
| F | With | 61 | 3.2 |
| c, | With | 29 | 1.9 |
| H | With | 157 | 7.3 |
| I | With | 150 | 5.7 |
| J | With | 57 | 1.9 |
| a | Without | 183 | 4.2 |
| b | Without | 79 | 2.1 |
| c | Without | 84 | 2.7 |
| d | Without | 65 | 3.8 |
| e | Without | 43 | 1.8 |
| f | Without | 90 | 2.7 |
| g | Without | 48 | 2.6 |
| h | Without | 76 | 2.9 |
| i | Without | 37 | 1.1 |
| j | Without | 38 | 1.3 |
| k | Without | 14 | 0.5 |
| l | Without | 25 | 1.0 |
| m | Without | 44 | 0.9 |
| n | Without | 87 | 3.0 |
| o | Without | 69 | 1.7 |
| p | Without | 85 | 3.2 |
| q | Without | 74 | 1.9 |
| r | Without | 111 | 4.2 |
| s | Without | 107 | 3.4 |
| t | Without | 0 | 0.0 |
| u | Without | 106 | 3.5 |
| v | Without | 152 | 3.9 |
| w | Without | 160 | 5.0 |
| x | Without | 128 | 3.3 |
| y | Without | 61 | 1.8 |
| z | Without | 106 | 2.8 |

**[Table 3]**

| Case No. | DVT/PE | sCLEC2 after surgery pg/mL | C2PACvalue |
|---|---|---|---|
| aa | Without | 65 | 2.3 |
| bb | Without | 102 | 3.2 |
| cc | Without | 57 | 1.6 |
| dd | Without | 129 | 3.9 |
| ee | Without | 57 | 1.4 |
| ff | Without | 73 | 1.6 |
| gg | Without | 90 | 2.1 |
| hh | Without | 82 | 2.8 |
| ii | Without | 134 | 4.7 |
| jj | Without | 199 | 4.2 |
| kk | Without | 57 | 2.3 |
| 11 | Without | 60 | 2.2 |
| mm | Without | 65 | 3.6 |
| nn | Without | 146 | 4.8 |
| oo | Without | 107 | 2.3 |
| pp | Without | 242 | 7.9 |
| qq | Without | 159 | 12.0 |
| rr | Without | 156 | 5.4 |
| ss | Without | 254 | 9.0 |
| tt | Without | 101 | 2.9 |
| uu | Without | 86 | 5.3 |
| vv | Without | 139 | 2.9 |
| Healthy 1 | | 66 | |
| Healthy 2 | | 63 | |
| Healthy 3 | | 10 | |
| Healthy 4 | | 51 | |
| Healthy 5 | | 31 | |
| Healthy 6 | | 8 | |
| Healthy 7 | | 29 | |
| Healthy 8 | | 39 | |
| Healthy 9 | | 68 | |
| Healthy 10 | | 37 | |
| Healthy 11 | | 155 | |
| Healthy 12 | | 51 | |
| Healthy 13 | | 85 | |
| Healthy 14 | | 73 | |
| Healthy 15 | | 19 | |

**[Table 4]**

| | Patients with DVT/PE complications | Patients without DVT/PE complications |
|---|---|---|
| Mean | 118.7 | 96.5 |
| Standard deviation | 52.3 | 53.9 |
| Median | 139.5 | 85.5 |

### INDUSTRIAL APPLICABILITY

As described above, by applying the method of the present invention for predicting the risk of thrombosis by measuring sCLEC2 in blood, the risk of thrombosis can be predicted in patients before they develop thrombosis, and appropriate treatment can be started promptly. Therefore, the method of the present invention for predicting the risk of thrombosis is applicable to a wide range of fields such as medicine and biology and is especially useful in the field of clinical testing.

## Claims

1. A method for assessing a risk of cancer-associated thrombosis in a perioperative period of a cancer patient, comprising the step of measuring a concentration of soluble CLEC2 in blood collected from the cancer patient.

2. The method according to claim 1, comprising:
(1) providing a sample from a patient who may have cancer-associated thrombosis, or a patient who has been diagnosed with cancer-associated thrombosis;
(2) determining a concentration of soluble CLEC2 in the sample; and
(3) correlating the soluble CLEC2 concentration with a presence or absence of cancer-associated thrombosis in the patient, or with likelihood of outcome.

3. The method according to claim 1 or 2, wherein the step of correlating the soluble CLEC2 concentration with a presence or absence of cancer-associated thrombosis in the patient, or with likelihood of outcome comprises a step of assessing whether the patient is at risk of cancer-associated thrombosis based on a change in the soluble CLEC2 concentration.

4. The method according to any one of claims 1 to 3, wherein at least one of coagulation and hemostasis-related marker is used in addition to the soluble CLEC2 concentration.

5. The method according to any one of claims 1 to 4, wherein a value obtained by dividing the soluble CLEC2 concentration by a platelet count is used instead of the soluble CLEC2 concentration.

6. A method for predicting efficacy determination of an antiplatelet agent in a method for assessing a risk of cancer-associated thrombosis in a perioperative period of a cancer patient, by providing samples derived from the cancer patient from preoperative period to 30 days postoperatively over time, and monitoring risk assessment continuously.

7. The method according to any one of claims 1 to 6, wherein the cancer is selected from the group consisting of pancreatic cancer, squamous cell carcinoma (lung, esophageal, cervix, and the like), mesothelioma, brain tumor, advanced cancer, and myeloproliferative disease.

8. The method according to any one of claims 1 to 7, wherein the cancer-associated thrombosis is Trousseau syndrome.
